# EUROPÄISCHE PATENTSCHRIFT

(11) **EP 0 886 491 B1**
(45) Veröffentlichungstag und Bekanntmachung des Hinweises auf die Patenterteilung: **28.05.2003**
(21) Anmeldenummer: 96938913.9
(22) Anmeldetag: 19.08.1996
(51) Int. Cl.: A61B 17/32, A61B 17/04, F16C 35/067, F16C 1/18

(54) **MOTORGETRIEBENES MEDIZINISCHES INSTRUMENT MIT ABWINKELBARER WELLE**
MOTOR-DRIVEN MEDICAL INSTRUMENT WITH FLEXIBLE SHAFT
INTRUMENT MEDICAL ACTIONNE PAR MOTEUR ET MUNI D'UN ARBRE POUVANT FORMER UN ANGLE

(30) Priorität: 18.08.1995 DE 19530478
(43) Veröffentlichungstag der Anmeldung: 30.12.1998
(73) Patentinhaber: Karl Storz GmbH & Co. KG, 78532 Tuttlingen (DE)
(72) Erfinder: STORZ, Karl, . (DE)
(74) Vertreter: Münich, Wilhelm, Dr.
(86) Internationale Anmeldenummer: DE9601537
(87) Internationale Veröffentlichungsnummer: WO97006736

(56) Entgegenhaltungen:
- EP-A- 0 634 146
- CH-A- 494 022
- DE-A- 3 122 061
- DE-A- 3 536 747
- DE-C- 44 898
- DE-C- 549 080
- DE-C- 838 938
- FR-A- 2 681 919
- US-A- 1 984 663

## Beschreibung

Die Erfindung bezieht sich auf ein medizinisches Instrument gemäß Anspruch 1.

Medizinische Instrumente des im Oberbegriff des Anspruchs 1 vorausgesetzten Standes der Technik nach EP-A-634 146 werden beispielsweise in Form sogenannter Sheaver eingesetzt. Derartige Instrumente weisen einen am proximalen Ende des Instruments angeordneten Motor auf, dessen Abtriebswelle über eine in dem Instrument angeordete Übertragungs-welle oder direkt mit wenigstens einem am distalen Ende angeordneten Eingriffselement, wie einem Messer, einem Abladierelement oder dergleichen verbunden ist.

### Stand der Technik

Die bekannten gattungsgemäßen Instrumente sind entweder so ausgebildet, daß der Motor koaxial zu der Welle in dem Instrument angeordnet ist, oder daß die Motorabtriebswelle und die Welle in dem Instrument einen bestimmten, vorgegebenen und nicht veränderbaren Winkel einschließen. Dabei ist in der Regel der Motor in dem Handstück angeordnet, an dem der Arzt das Instrument hält.

Welche der möglichen Anordnungen bevorzugt wird, hängt von persönlichen Vorlieben der Bedienungsperson sowie von dem jeweils auszuführenden Operationsvorgang ab.

Deshalb sind in der Vergangenheit sowohl Instrumente, bei denen das Handstück unter einem Winkel zum Instrument angeordnet ist, als auch Instrumente angeschafft worden, bei denen das Handstück und das Instrument koaxial angeordnet sind. Dies bedeutet einen vergleichsweise hohen Investitionsaufwand.

### Darstellung der Erfindung

Der Erfindung liegt die Aufgabe zugrunde, ein gattungsgemäßes medizinisches Instrument derart weiterzubilden, daß der Investitionsaufwand reduziert wird.

Eine erfindungsgemäße Lösung dieser Aufgabe ist im Anspruch 1 angegeben.

Bei der im Anspruch 1 angegebenen Lösung ist der Motor in an sich bekannter Weise lösbar mit der Welle verbunden. Zwischen Motorwelle und der im Instrument angeordneten Übertragungswelle kann ein Winkelstück eingesetzt werden.

Damit ist es möglich, den Motor sowohl koaxial an das Instrument anzuflanschen, als auch ihn mittels des Winkelstücks mit dem Instrument zu verbinden, so daß er unter einem Winkel angesetzt ist.

Ein Längenausgleich bei unterschiedlichen Bauformen des Motors und des Instruments wird erreicht wie folgt:

Hierzu ist das eine Ende einer ersten drehbaren Schraubenfeder an einer Aufnahme für die Motorabtriebswelle und das andere Ende an einem drehbar gelagerten Verbindungselement angebracht. Das drehbar gelagerte Verbindungselement ist über die biegsame Welle oder das Kardangelenk mit einer Aufnahme für die Übertragungswelle verbunden.

Bei der im Anspruch 2 angegebenen Lösung ist zwischen Motor und Übertragungsweile ein flexibles Winkelstück eingesetzt. Durch Einstellen dieses Winkelstücks kann der Motor sowohl koaxial zur welle als auch unter einem beliebigen einstellbaren Winkel angeordnet werden.

Im Anspruch 3 ist gekennzeichnet, daß der Motor in an sich bekannter Weise in einem Handstück des Instruments angeordnet ist. Hierdurch wird die Ergonomie des Instruments verbessert, da die Bedienungsperson das Instrument an einem Handgriff halten kann.

Die im Anspruch 2 gekennzeichnete Weiterbildung, bei der die Kraftübertragung durch eine biegsame Welle erfolgt, hat den Vorteil, daß sie nicht nur einfach und kostengünstig zu realisieren ist, sondern daß sie auch in einfacher Weise eine Einstellung des Winkels zwischen Motorabtriebswelle und Übertragungs-Welle erlaubt.

Bei medizinischen Instrumenten ist es erforderlich, daß das Instrument zur Reinigung und Sterilisierung zerlegt werden kann. Die im Anspruch 6 angegebene Weiterbildung vereinfacht den Zusammenbau des Instruments nach einer

Zerlegung: Hierzu ist eine zweite feststehende Schraubenfeder vorgesehen, die das Kardangelenk umgibt und ein Lagerelement, das distalseitig an dem Kardangelenk angebracht ist, in einen Konus drückt.

Im Anspruch 5 ist eine Ausgestaltung gekennzeichnet bei der in äußerst einfacher Weise die Einstellung zweier Winkel, beispielsweise 0° und 90° möglich ist:
Gemäß dieser Weiterbildung ist ein zweiteiliges Gehäuse vorgesehen, dessen beide Gehäusehälften gegeneinander zur Einstellung des Winkels zwischen Motor-Abtriebswelle und Übertragungswelle verdrehbar sind.

### Kurze Beschreibung der Zeichnung

Die Erfindung wird nachstehend anhand eines Ausführungsbeispiels unter Bezugnahme auf die Zeichnung näher beschrieben, in der zeigen
Fig. 1 ein Ausführungsbeispiel eines Winkelstücks,
Fig. 2 das in Fig. 1 dargestellte Winkelstück im gestreckten Zustand ohne Gehäuse.

### Darstellung des Ausführungsbeispiels

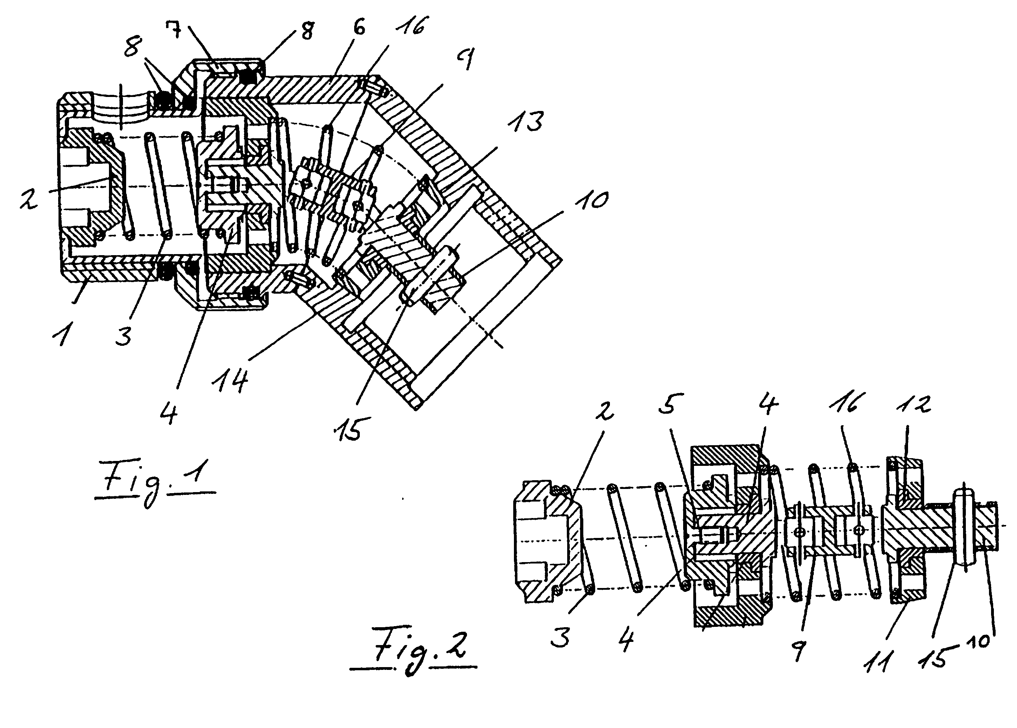

In den Fig. 1 und 2 ist lediglich das Winkelstück dargestellt. Das am distalen Ende des erfindungsgemäßen Instruments angeordnete Eingriffs-Element, wie ein Messer o. dgl. sowie der am proximalen Ende angeordnete Motor sind in an sich bekannter Weise ausgebildet - hierzu wird beispielsweise auf den Sheaver der Karl Storz GmbH & Co, Tuttlingen, DE verwiesen. Deshalb wird auf eine Beschreibung dieser Teile verzichtet.

Das in den Figuren 1 und 2 dargestellte Winkelstück weist eine in einem Grundteil 1 des Gehäuses drehbar gelagerte Aufnahme 2 für die nicht dargestellte Motorabtriebswelle auf. Die Aufnahme 2 ist über eine drehbare Schraubenfeder 3 mit einem Verbindungselement 4 verbunden, so daß eine Drehbewegung der Abtriebswelle des Motors auf das Verbindungselement 4 übertragen wird. Die Schraubenfeder 3 dient zum Längenausgleich, der aufgrund unterschiedlicher Bauformen des Motorteils und des Eingriffselements erforderlich ist. Mit 5 ist eine Befestigungsschraube bezeichnet.

Das Verbindungselement 4 ist in der einen Hälfte 6 eines zweiteiligen Gehäuses drehbar gelagert. Das zweiteilige Gehäuse ist mittels einer Überwurfmutter 7 mit dem Grundteil 1 verbunden. O-Ringe 8 dichten die Gehäuse fluiddicht gegenüber der Umgebung ab, Damit können die Teile für die Reinigung leicht auseinandergenommen werden.

Auf der distalen Seite des drehbar gelagerten Verbindungselements 4 ist die eine Seite eines Kardangelenk 9 angelenkt, dessen andere Seite mit einer Aufnahme 10 für die Übertragungswelle des - nicht dargestellten - Eingriffselements verbunden ist. Die Aufnahme 10 ist über ein Gleitlager 12 in einem Lagerelement 11 drehbar gelagert, das mittels eines Konus 13 in den zweiten Teil 14 des zweiteiligen Gehäuses eingesetzt ist. Mit 15 ist ein Zapfen bezeichnet, der als Verdrehsicherung zwischen der Aufnahme 10 und der Übertragungswelle des Eingriffselements dient.

Eine zweite feststehende Schraubenfeder 16 umgibt das Kardangelenk 9 und drückt das Lagerelement 11 in den Aufnahmekonus an dem zweiten Teil 14 des zweiteiligen Gehäuses.

Bei dem gezeigten Ausführungsbeispiel sind die beiden Gehäusehälften 6 und 14 fest miteinander verbunden. Es ist jedoch auch möglich, die beiden Gehäusehälften gegeneinan der zur Einstellung des Winkels zwischen Motor-Abtriebs welle und Antriebswelle des Eingriffselements verdrehbar zu gestalten. Damit sind beispielsweise die Winkel 0°und 45° einstellbar.

Vorstehend ist die Erfindung anhand eines Ausführungsbeispiels unter Bezugnahme auf die Zeichnung näher beschrieben worden, auf die zur Erläuterung aller nicht näher beschriebener Einzelheiten ausdrücklich verwiesen wird.

## Patentansprüche

1. Medizinisches Instrument, mit
- wenigstens einem am distalen Ende angeordneten Eingriffs-Element, wie einem Messer,
- einem am proximalen Ende angeordneten Motor mit einer Motorwelle,
- einer Übertragungswelle, welche die Motorwelle mit dem Eingriffs-Element verbindet, so daß das Eingriffs-Element gedreht wird,
- einem Winkelstück zwischen Motorwelle und der Übertragungswelle mit Aufnahme (2) für die Motorwelle
- einer lösbaren Verbindung zwischen Motor und der Übertragungswelle,
- eine biegsame Welle oder ein Kardangelenk (9) in dem Winkelstück, welche die Drehung der Motorwelle auf die in dem Instrument vorgesehene Welle überträgt,
**gekennzeichnet durch** die Kombination folgender Merkmale:
- zum Ausgleich unterschiedlicher Längen zwischen Motor und Eingriffs-Element aufgrund verschiedener Bauformen ist das eine Ende einer ersten drehbaren Schraubenfeder (3) an einer Aufnahme (2) für die Motorabtriebswelle und das andere Ende an einem drehbar gelagerten Verbindungselement (4) angebracht, so daß eine Drehbewegung der Motorwelle auf das Verbindungselement (4) übertragen wird, und
- das drehbar gelagerte Verbindungselement (4) ist über die biegsame Welle oder das Kardangelenk (9) mit einer Aufnahme (10) für die Übertragungswelle verbunden.

2. Medizinisches Instrument nach Anspruch 1,
**dadurch gekennzeichnet, daß** das Winkelstück flexibel ist, so daß die Motorwelle und die Übertragungswelle einen einstellbaren Winkel einschließen.

3. Instrument nach Anspruch 1 oder 2,
**dadurch gekennzeichnet, daß** der Motor in einem Handstück angeordnet ist.

4. Instrument nach einem der Ansprüche 1 bis 3,
**dadurch gekennzeichnet, daß** eine zweite feststehende Schraubenfeder (16) das Kardangelenk (9) umgibt und ein Lagerelement (11), das distalseitig an dem Kardangelenk (9) angebracht ist, in einen Konus (13) drückt.

5. Instrument nach Anspruch 1,
**dadurch gekennzeichnet, daß** ein zweiteiliges Gehäuse vorgesehen ist, dessen beide Gehäusehälften gegeneinander zur Einstellung des Winkels zwischen Motor-Abtriebswelle und Übertragungswelle verdrehbar sind.

6. Instrument nach einem der Ansprüche 1 bis 5,
**dadurch gekennzeichnet, daß** das Element zum Reinigen bzw. Sterilisieren zerlegbar ist.

## Claims

1. Medical instrument, comprising
- at least one surgical element such as a knife;
- a motor disposed at a proximal end and having a motor shaft;
- a transmission shaft connecting the motor shaft with the surgical element so that the surgical element is rotated;
- an angle-piece between the motor shaft and the transmission shaft, having a seat (2) for the motor shaft;
- a releasable connection between the motor and the transmission shaft;
- a flexible shaft or a cardan joint (9) in the angle-piece, which transmits the rotation of the motor shaft to the shaft provided in the instrument;
**characterized by** the combination of the following features:
- for compensating differences of distance between the motor and the surgical element due to differences of design, one end of a first rotatable helical spring (3) is attached to the seat (2) for the motor output shaft, and the other end to a rotatably supported connecting element (4), so that a rotary movement of the motor shaft is transmitted to the connecting element (4); and
- the rotatably supported connecting element (4) is connected via the flexible shaft or the cardan joint (9) to a seat (10) for the transmission shaft.

2. Medical instrument according to claim 1,
**characterized in that** the angle-piece is flexible, so that the motor shaft and the transmission shaft form an adjustable angle.

3. Instrument according to claim 1 or 2,
**characterized in that** the motor is disposed in a hand-piece.

4. Instrument according to any one of claims 1 to 3,
**characterized in that** a second fixed helical spring (16) surrounds the cardan joint and urges a bearing element (11) which is attached to the cardan joint (9) on the distal side into a cone (13).

5. Instrument according to claim 1,
**characterized in that** a two-part housing is provided, the two housing halves of which are rotatable with respect to each other for adjusting the angle between the motor output shaft and the transmission shaft.

6. Instrument according to any one of claims 1 to 5,
**characterized in that** the element can be disassembled for cleaning or sterilizing.

## Revendications

1. Instrument médical comprenant :
- au moins un élément opératoire disposé à l'extrémité distale, par exemple une lame,
- un moteur avec un arbre de moteur disposé à l'extrémité proximale,
- un arbre de transmission reliant l'arbre du moteur à l'élément opératoire de façon à faire tourner l'élément opératoire,
- un renvoi d'angle entre l'arbre du moteur et l'arbre de transmission avec un logement (2) pour l'arbre de moteur,
- une liaison amovible entre le moteur et l'arbre de transmission,
- un arbre flexible ou un joint de cardan (9), dans le renvoi d'angle, qui transmet la rotation de l'arbre du moteur à l'arbre prévu dans l'instrument,
**caractérisé par** la combinaison d'attributs suivante :
- pour compenser les différences de longueur dues aux formes de construction différentes entre le moteur et l'élément opératoire, l'une des extrémités d'un premier ressort cylindrique tournant (3) est montée dans un logement (2) destiné à recevoir l'arbre mené du moteur et l'autre extrémité est montée dans un élément de liaison (4) supporté en rotation de façon à transmettre un mouvement de rotation de l'arbre du moteur à l'élément de liaison (4), et
- l'élément de liaison (4) supporté en rotation est relié par l'intermédiaire de l'arbre flexible ou du joint de cardan (9) à un logement (10) destiné à recevoir l'arbre de transmission.

2. Instrument médical selon la revendication 1, **caractérisé en ce que** le renvoi d'angle est flexible de façon que l'arbre du moteur et l'arbre de transmission fassent un angle réglable entre eux.

3. Instrument selon la revendication 1 ou la revendication 2, **caractérisé en ce que** le moteur est disposé dans une pièce à main.

4. Instrument selon l'une des revendications 1 à 3, **caractérisé en ce qu'**un deuxième ressort cylindrique fixe (16) entoure le joint de cardan (9) et comprime dans un cône (13) un élément d'appui (11) disposé distalement par rapport au joint de cardan (9).

5. Instrument selon la revendication 1, **caractérisé en ce qu'**il est prévu un boîtier en deux parties dont les deux moitiés peuvent tourner l'une dans le sens contraire de l'autre pour ajuster l'angle entre l'arbre mené du moteur et l'arbre de transmission.

6. Instrument selon l'une des revendications 1 à 5, **caractérisé en ce que** l'élément peut être démonté pour le nettoyage ou la stérilisation.
